# EUROPEAN PATENT SPECIFICATION

(11) **EP 4 646 688 B1**
(45) Date of publication and mention of the grant of the patent: **11.11.2026**
(21) Application number: 23817704.2
(22) Date of filing: 30.11.2023
(51) Int. Cl.: G06T 7/00

(54) **METHOD FOR MODELLING AND ASSESSING THE EVOLUTION OF SIGNS OF AGEING IN A USER**
VERFAHREN ZUR MODELLIERUNG UND BEURTEILUNG DER ENTWICKLUNG VON ANZEICHEN DER ALTERUNG BEI EINEM BENUTZER
PROCÉDÉ DE MODÉLISATION ET D'ÉVALUATION DE L'ÉVOLUTION DE SIGNES DE VIEILLISSEMENT CHEZ UN UTILISATEUR

(30) Priority: 06.01.2023 FR 2300171
(43) Date of publication of application: 12.11.2025
(73) Proprietor: L'OREAL, 75008 Paris (FR)
(72) Inventor: BOKARIS, Panagiotis-Alexandros, 92110 Clichy (FR); DESPOIS, Julien, 93400 Saint Ouen (FR); JOUNI, Hussein, 93600 Aulnay-sous-Bois (FR); WU, Jun, Shanghai 201206 (CN)
(74) Representative: L'Oreal
(86) International application number: PCT/EP2023/083794
(87) International publication number: WO 2024/146718

(56) References cited:
- EP-A1- 3 699 811
- EP-A2- 1 643 448
- EP-B1- 2 174 296
- US-A1- 2021 407 153

## Description

The present invention relates to a method for modelling, predicting and assessing the evolution of signs of ageing in the skin of a user, and to a corresponding computer-implemented system for virtually simulating said evolution on an image of the user.

Many people are interested in knowing the extent to which their skin is likely to change over time and, notably, to show relatively pronounced visible signs of their age. Signs of skin ageing is understood to mean wrinkles or brown spots, for example. This knowledge simply can be out of curiosity, or with the aim of taking preventive measures, notably by applying a cosmetic or therapeutic treatment, or even by changing their lifestyle habits whenever possible.

Indeed, the presence of wrinkles or spots on the skin, and in particular on skin surfaces that are generally uncovered and therefore visible, such as the face or hands, often poses aesthetic problems that, depending on the individual, can be more or less difficult to live with.

The evolution of such signs of ageing over time depends on many factors, both internal, specific to the individual (skin type, genetics) and external (pollution, UV exposure, smoking), which can be difficult to understand and make such projections complex.

One method that was still used relatively recently involved using atlases comprising one or more sets of photos for different skin types at different stages in time (*Skin Aging Atlas* published in 5 volumes: Roland Bazin, Frédéric Flament, Huixia Qiu: Skin Aging Atlas. Volume 5, Photo-aging Face & Body. October 2017*;* Roland Bazin, Frédéric Flament, Virginie Rubert: Skin Aging Atlas. Volume 4, Indian Type. June 2014*;* Roland Bazin, Frédéric Flament, Franck Giron: Skin Ageing Atlas. Volume 3, Afro-American type. May 2012*;* Roland Bazin, Frédéric Flament: Skin Aging Atlas. Volume 2, Asian type. November 2010*;* Roland Bazin: Skin Aging Atlas Volume 1, Caucasian Type. October 2007*).*

One of the main aims of these atlases is to allow objective assessment of facial ageing. These books study and thus systematically establish a characterisation and a classification of the skin according to age and ethnic type, and allow a severity score (generally ranging from 0 to 9) to be assigned to different areas of the face (pore opening, glabellar wrinkles, crow's feet, sagging neckline, etc.). The classifications also can be used together in order to assess the overall ageing of the face.

These atlases are mainly reserved for professionals and are mainly intended to allow objective assessment of the results of "anti-ageing" treatments aimed at blending or masking these signs (localised treatments such as the injection of botulinum toxin into crow's feet wrinkles, for example, but also for more general treatments of the lifting or peeling type, for example) by comparing before/after severity scores.

The development of computer processing means and the deployment of artificial intelligence methods have allowed these assessments to be automated. For example, in January 2019, VICHY was able to offer a tool called SKINCONSULT AI allowing consumers to acquire a digital diagnosis of their skin based on a photographic portrait.

This tool is based on an artificial intelligence algorithm developed by MODIFACE and trained using a bank of L'OREAL images that were notably used to form the aforementioned atlases. For further details, please refer to application WO 2020/113326 A1.

Thus, based on a photographic portrait, the tool assesses seven signs of ageing: wrinkles under the eyes, lack of firmness, fine lines, lack of radiance, pigmentation spots, deep wrinkles and pores, and can offer the user product recommendations.

Although easily used by the general public, this tool, like the atlases, provides an assessment at a given moment and does not allow any assessment of how these signs of ageing are likely to evolve over time.

Artificial intelligence computing techniques have also been used in a number of applications in methods designed to provide the user with a prediction of the potential evolution of their face over time, notably by modifying their photo in order to simulate their possible appearance at a given age.

Examples of such methods are notably described in documents EP 1298562 A1, US 4276570, FR 2875930 A1 and in particular EP 3699811 A1 and US 2018/276869, for further useful reference.

Document EP 1298562 describes a method comparing ageing data acquired on a user at a first instant and at a second later instant (for example, 6 months later) in order to extrapolate a subsequent evolution (for example, 18 and 66 months later). Such a method is limited to a few months and is difficult to use for simulating a possible evolution over 10, 15 or 20 years.

A similar approach is used in document US 4276570 in that a photograph of a person at two different ages is used to transfer the detected evolutions to another person to be aged virtually. A similar method is described in the document by Lanitis A., et al., entitled, 'MODELING THE PROCESS OF AGEING IN FACE IMAGES', Computer Vision, 1999, The Proceedings of the Seventh IEEE International Conference on Kerkyra, Greece, 20-27 Sept. 1999, Los Alamitos, CA, USA, IEEE Comput. Soc., US, Vol. 1, 20 September 1999, pages 131-136.

Document FR 2875930 relates to a method for predicting the appearance of an external portion of the human body as a function of time and/or of a treatment, in which at least three images are generated, with these images corresponding to different scores of at least one aspect parameter as a function of time and/or the treatment, with the variation of at least this aspect parameter on said images being non-linear. Preferably, the variation in said aspect parameter occurs according to an evolution law determined on the basis of observations of this aspect parameter carried out in a reference population. The predicted evolution is therefore not actually personalised and simply projects different severity scores of an ageing marker onto a user.

In order to better personalise the prediction of the evolution of the signs of ageing, application EP 3699811 A1 aims to integrate data on the lifestyle and habits of the user, notably in order to take into account several external ageing factors such as exposure to sunlight, sleep, smoking, etc. It is stated that machine learning means are implemented to predict the possible evolution of ageing. However, few details are provided and the application merely lists a set of potentially usable learning models.

In reality, it is extremely difficult, if not impossible, to conventionally train an artificial intelligence engine to predict the evolution over time of the visible signs of ageing over a period of several years. Indeed, it is difficult to contemplate having sufficient training data (and notably a "ground truth" for each parameter) over such a time scale.

It is therefore generally necessary to resort to additional theoretical modelling and to use average evolutions over populations of a given age.

This is notably provided in document US 2018/276869, which makes provision for detecting an ethnic typology of the subject and applying a corresponding average ageing model. Such a method does not allow personalisation and personalised external ageing factors to be taken into account.

The viral application "FaceApp" is thus known, which uses a photographic self-portrait to generate an image of how the user is likely to look at an older age. This application uses a set of transformations that are applied to various characteristic elements of the face, while attempting to preserve personal characteristics and traits as much as possible. The transformations are acquired by training artificial intelligence engines of the cGAN type (https:/analyticsindiamag.com/the-ai-behind-faceapp/ & https:/iq.opengenus.org/face-aging-cgan-keras/) on the basis of defined age groups.

For further details on such a technique, please refer to the article by Antipov, G.; Baccouche, M.; Dugelay, J.-L., entitled, 'FACE AGING WITH CONDITIONAL GENERATIVE ADVERSARIAL NETWORKS', Proceedings of the 2017 IEEE International Conference on Image Processing (ICIP), Beijing, China, 17-20 September 2017; pages 2089-2093 (https:/doi.org/10.48550/arXiv.1702.01983) and the article by Xinhua Liu, entitled, 'BIDIRECTIONAL FACE AGING SYNTHESIS BASED ON IMPROVED DEEP CONVOLUTIONAL GENERATIVE ADVERSARIAL NETWORKS', MDPI Information 2019, 10, 69; doi:10.3390/info10020069.

The models thus acquired allow statistical transformations to be applied that remain extremely limited in terms of personalisation and generally do not take into account parameters relating to the lifestyle of the user, notably since increasing the number of conditions would drastically reduce the amount of usable training data to the point of making it insufficient.

The "ChangeMyFace" application (https:/changemyface.com/) proposes taking into account parameters relating to the lifestyle of the user. Possible parameters include diet, alcohol consumption, level of physical exercise, stress levels, exposure to a polluted environment, exposure to sunlight, amount of sleep, cigarette consumption. However, no information is provided relating to how these parameters are taken into account in order to influence the modification of the photograph of the user.

EP2174296B1 discloses a method that predicts and visualizes skin ageing. It uses wrinkle severity score and an aging model to generate a wrinkle simulated image.

EP1643448A2 involves generating three images related to different grades of appearance parameters according to time and/or treatment. The appearance parameters are varied on the images in a non-linear manner according to an evolution law that is determined from observations of the parameters in a reference population.

The thesis by Farnaz Majid Zadeh Heravi entitled, 'THREE-DIMENSION FACIAL DE-AGEING AND AGEING MODELING: EXTRINSIC FACTORS IMPACT', Signal and Image processing, Université Paris-Est, 2019 (https:/tel.archives-ouvertes.fr/tel-03467715) aims to take into account external factors such as exposure to sunlight, cigarette consumption, air pollution, as well as intrinsic personal factors, such as the ethnicity of the person or even their hormone levels. The aim of this work is to model the impact of these parameters in order to incorporate them into the rendered image generator. However, this can be particularly complex and increases the number of parameters to be integrated into the model.

Consequently, a requirement exists for developing a method and a system for improving both the accuracy of the evolution prediction and the possibility of personalising this evolution prediction for the user. The method also must remain relatively simple so that it can be easily implemented via portable personal computer terminals.

To this end, the present invention proposes a method as defined by claim 1.

The method is fully or partly implemented by a computer.

The term "generally showing a sign of ageing" is understood to mean that the targeted body area is an area in which the sign of ageing considered in the general population appears and manifests itself, even if said sign is not yet specifically present on the user undergoing the present method. Thus, even if the user is not yet old enough to have visible crow's feet wrinkles, the area in which they occur is well known and the degree of severity will simply be zero or one (or a minimum of the scale). The same applies to the other aforementioned visible signs of ageing. Of course, the image of the considered body area can be acquired from an image covering a larger body area encompassing the body area of interest, which then can be isolated by a segmentation or classification method based on body markers (for example, a computer image analysis can be used to isolate the body area in which crow's feet wrinkles appear, based on identification of the pupil and the edge of the eye).

In particular, the visible sign of ageing is selected from among: the opening of the skin pores, glabellar wrinkles, crow's feet wrinkles, neck sagging, wrinkles under the eyes, the firmness or sagging of the skin, the presence of fine lines, the brightness of the complexion or skin, the presence of pigmentation spots.

A severity score or degree of severity is understood to mean a numerical value forming part of an ordered scale. The scale can include a minimum value, notably zero or one. The scale can also include a maximum value. As mentioned above, the conventional atlases set a maximum value of nine. The evolution of the degrees on the severity scale can be expressed as whole numbers (0, 1, 2, 3 ..., 9) or decimal numbers, notably in half degrees (0, 0.5, 1, 1.5, ...).

Thus, by assigning a severity score to a sign of ageing, it is possible to use this score to perform simple computations, notably additive computations, as a function of values assigned to one or more ageing factors according to their impact on said sign of ageing, notably by incrementing or decrementing the severity score initially assigned according to the lifestyle habits of the user. The modified severity score then corresponds to a projection over time of the evolution of the considered sign of ageing. For example, for a user with skin whose degree of wrinkle severity has been assessed as 5, it will be possible to project a degree of severity in 10, 15 or 20 years by simply adding or subtracting degrees according to the values, preferably personalised values, given to the various considered ageing factors.

The modified severity score then can be used as input data for an image generator configured to transform the initial image as a function of an input parameter corresponding to a severity score and thus generate a modified image presenting the considered sign of ageing with the desired modified degree of severity.

Thus, in an advantageously complementary manner, the method comprises an additional step of generating a modified image of the body area of the user with a visible sign of ageing corresponding to the modified severity score. Advantageously, the modified image can be displayed on a screen for presentation to the user.

The modified severity score may or may not be displayed, separately or together with the modified image, for presentation to the user.

The initial severity score and/or the initial image also can be displayed for presentation to the user in order to compare any evolution. The severity scores (initial and modified) and/or the images (initial and modified) can be displayed separately or side-by-side to facilitate the comparison.

Preferably, the method comprises the use of at least one ageing factor that is independent of (or not specific to) the user, such as the time and notably the duration over which the evolution of the considered sign of ageing is intended to be projected. This duration is preferably greater than 1 year, preferably greater than 6 years, or even greater than 10 years. Preferably, a 10, 15 or 20 year projection will be sought.

Thus, for example, a duration of 15 years can be considered to increase the severity of wrinkles by two degrees (+2), or, for the same example, the severity of skin wrinkles will reach a value of 7.

Preferably, the method comprises the use of at least one ageing factor dependent on (or specific to) the user. The method then advantageously comprises an additional step of receiving data corresponding to personalised values adapted to the user for all or some of said ageing factors that are dependent on the user. In the absence of data for certain factors, default values can be provided.

Advantageously, the personalised values can correspond to a current state of the user.

In a complementary or optional manner, the method can be implemented with personalised data corresponding to a modified state of the user (counterfactual approach, for example, if a smoking user stopped smoking, or if a user who did not use a moisturising cosmetic product adopted such a product).

The various modified severity scores and, if applicable, the corresponding modified images, can be displayed and presented to the user for comparison according to the changes made in the values of the considered ageing factors.

According to a first embodiment, the various ageing factors have a limited number of possible values, and preferably two or three or even four possible values at most. The proposed values will advantageously reflect an intensity of the presence of the considered ageing factor in the user.

According to a second embodiment, all or some of the various ageing factors have possible values, each associated with a weighting factor specific to the user. Thus, for example, it is possible to offer the user extreme values and ask them to associate weighting coefficients with them, allowing them to represent an intermediate intensity that is specific to them. These weighting coefficients will be reported and used to weight the impacts and variations determined for each extreme value so as to acquire an intermediate impact adapted to the user.

According to a particular embodiment, the factor independent of the user, and in particular the time, is set and is not personalised to the user. Alternatively, its value can be modified, notably by the user or an operator. However, particularly for the time factor, it is preferable to use a non-modifiable set duration. Indeed, the impacts established for ageing factors specific to the user (each factor or pooled between several factors) can also take into account a time factor, and implementing a modifiable time factor would require numerous resources to adapt said impacts of factors specific to the user, not only as a function of the values given by the user to these factors, but therefore also as a function of the intended duration for the projection.

According to one embodiment, the severity score is modified by applying a computer model configured to determine at least one variation in the severity score as a function of the values assigned to the ageing factors, with said variation being added to the initial severity score assigned to the considered sign of ageing.

Preferably, the method comprises an additional step of receiving information corresponding to a skin type of the user and applying a corresponding adapted computer model. In particular, a model of the variation or the evolution of the sign of ageing can be provided for each ethnic type, namely, notably, an evolution model for Caucasian skin, an evolution model for African skin and an evolution model for Asian skin. The skin type of the user can be provided manually or can be established from an image analysis step aimed, for example, at determining a skin colour parameter, as notably can be achieved in applications for determining foundation. This step is preferably carried out on the image data received for the body area for which the evolution of the signs of ageing are to be predicted. Similarly, provision also can be made to take into account data relating to the gender of the user and/or their age (or membership of an age group), each of which can be entered manually or can be the result of an image analysis assessment process for applying a model corresponding to the considered criterion.

According to an advantageous embodiment, it is possible to apply several models, notably designed for various types of users, and to weight the results. Such a method is particularly useful for the age of the user. Indeed, the evolution models will generally be designed for different age ranges of the user (it would be difficult to design one model per age). However, it would be simplistic to strictly apply the model of the age range to which the user belongs, with the risk of underestimating or overestimating their evolution, notably depending on whether the age of the user is relatively close to a lower limit or an upper limit. Thus, according to an alternative embodiment, the method comprises an additional step of acquiring, for example, via an interpolation function, one or more weighting coefficients to be assigned to the results of one or more predictive models to be applied according to the typology of the user. By applying each weighting coefficient to the result of each model, it is possible to acquire the final variation to be applied to the initial degree of severity.

Advantageously, this step is carried out for the age of the user, with the step aiming to acquire, as a function of the age of the user, weighting coefficients to be applied to different predictive models established by age brackets. The predictive models (all or at least those for which a non-zero or significant weighting coefficient is returned) are applied to the initial degree of severity and the acquired results are weighted with the coefficients determined by the interpolation function in order to acquire the modified degree of severity.

According to a particular embodiment, the computer model is a probabilistic model returning at least one variation in the severity score and an associated probability. Preferably, the computer model returns several variations in the severity score, each with an associated probability. Thus, the method can determine several modified severity scores, with each modified severity score corresponding to the initial severity score to which a variation has been added. Each modified severity score is associated with the probability corresponding to the added variation. Modified images corresponding to each modified severity score can be generated with a view to being displayed for presentation to the user. Preferably, only a modified image corresponding to the variation associated with the highest probability will be generated. Alternatively, an average modified severity score can be determined by weighting the variations with their associated probabilities.

Advantageously, the probabilistic computer model is a Bayesian network, and in particular a causal Bayesian network. Advantageously, the Bayesian network can be constructed from expert knowledge implementing an elicitation method and/or causal inference techniques.

The variation in the degree of severity determined by the model can result from computations carried out on impacts determined for one or more ageing factors depending on the value assigned thereto. In particular, an initial impact can be determined for each considered ageing factor, then the impacts advantageously can be pooled by grouping together several ageing factors. Advantageously, the ageing factors will be grouped into different categories, for example, beneficial factors, degrading factors, intrinsic factors, extrinsic factors and notably environmental factors, in particular in order to take into account any synergies between said factors, if applicable. The impacts of the various ageing factors according to their value can be determined empirically and/or theoretically. Depending on the models used, the impacts can be single-factor or multi-factor impacts.

Thus, if the user is a regular smoker, this evolution can be worsened by further increasing the severity of skin wrinkles by one or two degrees. It could be determined that a user who is a regular smoker in 15 years time risks having skin with a degree of wrinkle severity of 8 (initial severity of 5, +2 for a time factor set to 15 years, +1 for the "smoker" factor with a "yes" value).

Based on the projected degree of severity, it is advantageous to modify the initial image accordingly in order to render, for the user, a projected image of their skin in 15 years time.

Advantageously, the method is implemented on image data of a body area showing several visible signs of ageing, with a severity score being assigned to each sign of ageing according to this method, notably by applying a computer model dedicated to the evolution of each sign of ageing. A single modified image for all the signs of ageing, each with its associated modified severity score, is generated and, if applicable, displayed for presentation to the user.

Advantageously, assigning an initial degree of severity to the considered visible sign of ageing is carried out by image analysis, with or without prior segmentation of an area of interest.

In particular, the degree of severity is assigned by a trained artificial intelligence engine, such as SKIN CONSULT described above. Alternatively or additionally (see FR22/04419 not yet published), image analysis can be used to measure characteristic physical parameters from optical properties of the area of interest. The characteristic physical parameters can be, for example, the number, the density, the length and/or the depth of wrinkles (for example, by analysing contrast in a known manner), the colour or the contrast of areas of skin, as well as the colour, the number, the surface and/or the density of pigment spots, for example. The values of the measured physical parameters then can be used to assign a degree of severity to the considered sign of ageing.

Advantageously, the method comprises a prior step of acquiring the digital image of the body area. Preferably, the body area is the face.

Preferably, the image of the body area of the user is acquired under standardised conditions, and in particular under standardised illumination conditions, notably under D65 lighting conditions. Otherwise, notably in the case of self-portraits or selfies taken by the user themselves using a mobile terminal or smartphone, an image correction/ normalisation algorithm can be applied before the initial degree of severity is assigned by the image analysis algorithm.

In order to render a projected image to the user corresponding to the same image capture conditions, inverse transformations obviously can be applied to the modified image before displaying and before presentation to the user.

The present invention also relates to a system for implementing the present method, in particular a computer system for implementing said method on a computer.

In particular, the system comprises at least one first data input configured to receive the data corresponding to a set of pixels of a digital image and a second data input configured to receive at least one value of an ageing factor.

Preferably, the system comprises a digital photograph acquisition device communicating with the first data input.

Preferably, the system also comprises a human-machine interface, notably a keyboard or a touch screen connected to the second data input and allowing a user or an operator to enter the one or more values of the one or more ageing factors.

The system also comprises at least one processor configured to assign, from said received pixels, a severity score of at least one visible sign of ageing.

In accordance with the present application, the processor is also configured to modify said severity score as a function of the value of the received ageing factor, and to return said modified severity score.

Advantageously, in addition, the system comprises an image generator configured to generate, from the received image, a modified image presenting the modified degree of severity. The system further preferably comprises a screen for displaying and presenting to the user or operator all or some of the generated, and optionally received, information (notably for comparison purposes), namely the severity scores (assigned to the received and modified image) and the received and modified images.

The present invention will be better understood upon reading the following detailed description with reference to the appended drawings, in which:
[Fig.1] is a schematic representation of the main steps of the method that is the subject matter of the present application;
[Fig.2] is a schematic representation of a Bayesian network applied to determine a modified severity score of a visible sign of ageing.

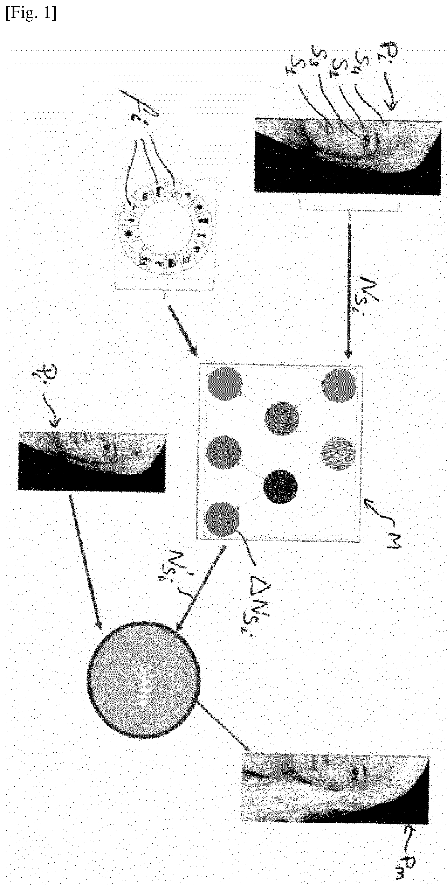

As shown in [Fig.1], an aim of the method according to the present application is to model, predict and assess the evolution of signs of ageing in a user and, if applicable, to generate a virtual image of their likely appearance in several years.

To this end, the method uses an image or photo *Pi* of at least one body area of a user that generally shows a visible sign of ageing. In this case, the image *Pi* is a photographic portrait of the face of the user, notably comprising an area of nasolabial wrinkles *S1*, an area of crow's feet wrinkles *S2,* an area of wrinkles and bags under the eyes *S3* and an area of forehead wrinkles *S4.*

The method generates a modified image *Pm* of the user as a function of various ageing factors *fi* likely to affect the evolution of the signs of ageing present on the initial image *Pi,* with said ageing factors notably including (all or some): the amount of sleep, the use of cosmetic products, sporting activity, exposure to sunlight, alcohol consumption, facial expressions, body mass index.

Thus, in a first step, a photographic portrait image is taken of the user. Advantageously, the photograph is taken using a high-definition digital camera, preferably in a standard lighting environment. Alternatively, the image can be acquired using a mobile terminal such as a smartphone or tablet computer comprising an integrated camera. The digital photograph then can be processed at a later date, notably to correct the exposure, the colours, etc.

The image data is then processed in order to identify and analyse the areas of the body that generally show signs of ageing, with a view to assigning an initial severity score to said sign of ageing.

As mentioned above, each body area of interest can be identified using one or more computer vision algorithms, and notably using artificial intelligence algorithms trained to this end on the basis of a photographic portrait. The detection and delimitation of each area of interest advantageously can occur based on the detection of body markers and in particular of facial markers.

A severity score *N_{Si}* for the sign of ageing considered for each body area of interest is then assigned. The severity score is advantageously determined and assigned by an image analysis algorithm and in particular by an artificial intelligence algorithm trained to this end.

For further details of such a method, please refer to the aforementioned application WO 2020/113326 A1. Of course, the present method is not limited to the implementation of such a system, and any method, notably a computer-implemented method, for identifying and segmenting a body area can be contemplated. The body area thus segmented can be noted on a severity scale by any means.

In accordance with the method that is the subject matter of the present application, the severity score assigned to each sign of ageing in each body area of interest is modified as a function of values given to the ageing factors *fi* that are taken into account.

Thus, at the same time as the image data is received, values are received for each of the considered ageing factors *fi.* At least some of the received values are adapted and personalised to the user according to their lifestyle habits. These values can be acquired via a questionnaire and/or entered manually via a computer interface.

It should be noted that not all the ageing factors *fi* are necessarily relevant for each considered sign of ageing and that an evolution model dedicated to a sign will use the values assigned to the factors *fi* considered relevant for its evolution. It is known, for example, that the evolution of pigmentation spots fundamentally depends on exposure to sunlight. The evolution model applied for assessing the evolution of this sign of ageing will therefore use the values assigned to the relevant *fi* factors, even if other factors have been requested from the user, notably for the purpose of assessing the evolution of other signs of ageing for which they would be relevant.

Preferably, the ageing factors propose a limited number of possible values (modalities). In particular, the possible values represent an intensity and/or frequency of exposure to said factor. Some factors can be Boolean, i.e., have two possible values of the YES / NO; 0 / 1; type, etc. Interestingly, other factors can have three or even four possible values so as to allow an intermediate or average intensity value to be taken.

According to a first embodiment, the ageing factors accept only one input selected from among the possible values. According to a second embodiment, one or more ageing factors accept several inputs from among the proposed possible values, with each input being associated with an intensity parameter or a weighting factor determined for the user. This type of operation allows better consideration of intermediate states, the impact of which can be difficult to assess.

Thus, for example, for an ageing factor such as facial expressivity, an expert will probably be able to easily assess the impact on ageing for extreme situations, namely hyper-expressivity or low expressivity. However, "average" expressivity will be almost impossible to assess and model. According to this embodiment, the user is asked to enter a personalised weighting coefficient for each proposed value ("hyper-expressivity" and "low expressivity"). Thus, instead of receiving a value corresponding to a deterministic state, it is possible to consider weighted values.

Using the example of facial expressivity, a user could thus consider themselves to be 30 % "hyper-expressive" and 70 % "low expressive". These weighting coefficients are reported and used to determine the impact of the corresponding ageing factor on the basis of the impacts established for the proposed extreme deterministic values. The human-machine interface used to gather these values and weightings is configured accordingly to allow this data to be received and processed.

Studies have shown, for example, that normal/neutral expressivity has no impact (impact equal to 0) on the acceleration of the marked character of ageing expression wrinkles. On the other hand, studies have shown that hyper-expressivity had a significant impact on the acceleration of the marked character of expression wrinkles (for example: 15 % probability of a 3-degree worsening in severity at 15 years, 30 % probability of a 2-degree worsening, 40 % of a 1-degree worsening, and 15 % of no impact). The weightings previously received from the user for these values corresponding to determined extreme states are then used to determine an impact probability table adapted to the user, as will be explained below.

A similar implementation can be implemented for ageing factors such as sleep quality, stress level, exposure to pollution, etc.

According to one embodiment, the factors *fi* form input variables (modulators) for a computer model *M* configured to determine at least one variation in the severity score *ΔN_{Si}* as a function of the values entered (evidence or observed nodes) for these factors *fi,* with the determined variation being intended to be added to the initial severity score *N_{Si}* assigned to the considered sign of ageing so as to acquire a modified severity score *N'_{Si}.* All the evidence forms a scenario corresponding to a simulated or actual state of the user.

In particular, the computer model *M* implements a causal Bayesian network, some preferred structural features of which are provided hereafter.

For each input variable corresponding to an ageing factor *fᵢ*, the model presents a dependency or causal link to a node defining an impact *Ii* representing a variation in the degree of severity for the considered factor. The impact *Iᵢ* assumes the form of a table of conditional probabilities associating each modality of the factor *fi* with a variation in the degree of evolution. The probability tables forming the impacts *Ii* of each factor particularly can be acquired by elicitation from expert knowledge and/or experimental data.

Preferably, the Bayesian network structure comprises only one level of such elicited impact nodes.

The impact nodes *Ii* are causally linked to pooled downstream nodes *Aij* aimed at grouping together the probabilities and variations by degree of severity of several factors until a total final variation *ΔN_{Si}* can be determined. The factors grouped in pooled nodes also can be determined by elicitation, and notably grouped by type of factor (external, internal, linked to the same externality, etc.).

Notably, factors with a negative impact can be grouped separately from factors with a positive impact, in one or more steps, in order to determine a node.

Each pooled node *Aij* is annotated with a table of conditional probabilities associating the variations in the degree of evolution that can result from the association of these factors. Various operations can be carried out in order to acquire the possible variations, and it is possible to retain, for example, the minimum variation between the parents, the maximum variation between the parents, or a sum of the variations between the parents, bearing in mind that it is preferable for such a sum to be restricted, from above and/or below. Thus, for example, it can be considered that the variation resulting from smoking and exposure to sunlight cannot exceed +2. The associated probabilities are computed accordingly, notably by applying Bayes' theorem. The nature of the operation to be applied can be determined by experts.

A final pooled node BT corresponds to the final total variation to be applied to the initial determined degree of severity. This node is acquired from the progressive pooling of all the considered factors and an impact node T not specific to the user, representing residual impacts or impacts without a known and/or quantifiable cause, and notably simply resulting from the passage of time. The operation that is applied is a sum of the variations, which is limited or restricted by the highest variation from among the worsening modalities of the considered factors.

In addition, the sum of the variations for determining the last pooled node BT is also restricted to be strictly greater than zero (with a negative variation implying rejuvenation).

Of course, other Bayesian network structures can be contemplated. However, such a model has the advantage of remaining simple to interpret (with the prediction being the simple result of an initial state to which the impacts of various factors/modulators are added). It is also a "white box" type model, i.e., the computations are known.

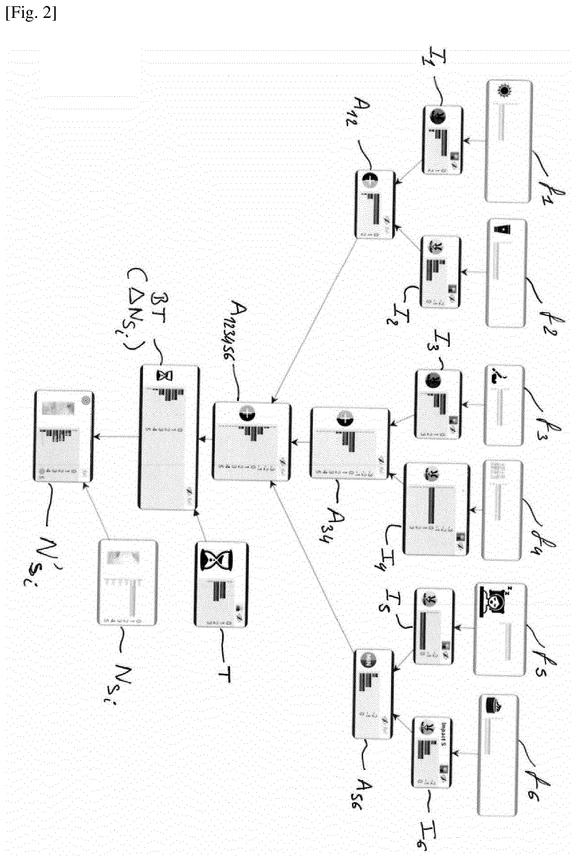

[Fig.2] shows an example of an application for determining an evolution of a nasolabial wrinkle *S1* of a user.

As described above, the first step involves assigning an initial severity score *N_{S1}* to the considered sign of ageing. In this case, the severity score is 1 on a scale of 0 to 5.

At the same time, the user indicates personalised values for various ageing factors likely to worsen the development of the nasolabial wrinkle.

In this case, the user indicated the following personalised values:
- *f1* - chronic exposure to sunlight (photoexposure): "medium" from the following choices: "little or no"; "low"; "medium"; "high";
- *f2* - application of a photoprotection product: "systematic" from the following choices: "never/rarely"; "recreational"; "systematic";
- *f3* - smoking: "between 10 and 20 packets per year" from the following choices: "less than 10"; "10 to 20"; "more than 20";
- *f4* - face shape: "neutral" from the following choices: "short and wide"; "neutral"; "long";
- *f5* - sleeping on the back: "no" from the following choices: "yes" or "no";
- *f6* - application of skin care products: "moisturisers - anti-ageing" from the following choices: "none"; "daily moisturiser"; "moisturisers and anti-ageing".

A corresponding impact *I1* ... *I6* is determined for each factor. As mentioned above, this impact is particularly determined on the basis of expert knowledge through elicitation. Each impact indicates the probability of a given variation in the degree of severity depending on the value of the considered factor.

Thus, for the factor *f1* representing the photo-exposure of the user, the highest probability is that this "average" exposure will have no significant long-term impact (zero variation). Of course, there is also a lower probability of worsening by 1 degree and an even lower probability of worsening by 2 degrees.

A probability table is also determined for the factor *f2* representing the application of a photoprotection product. For the considered user, the application of such a product is "systematic" and the most likely result is thus a zero impact on the degree of severity of their nasolabial wrinkle. As with the factor *f1,* there is a lower probability of an improvement of one degree (variation -1) and an even lower probability of an improvement of two degrees.

The impacts *I1, I2* of factors *f1* and *f2* are then pooled into a pooled node *A₁₂* representing the general impact of the sun and being made up of a probability table for each possible variation in the degree of severity. In this case, the variations are added and are greater than or equal to 0. The probabilities of each variation are computed accordingly.

A similar procedure is used for the other factors *f3* to *f6.* The impacts *I3, I4* of factors *f3* and *f4* are pooled together, while the impacts *I5, I6* of factors *f5* and *f6* are pooled together and are considered to be factors with improving impacts.

All the pooled nodes *A12, A34, A56* are in turn pooled into a dependent node *A123456* accumulating all the probable variations for the considered factors *f1* to *f6.*

A final variation *BT* is determined by applying a probability table for a time factor *T* representing the residual impacts at 15 years. As shown, at 15 years, the probability of evolution of the nasolabial wrinkle is two degrees, only slightly more likely than a variation of 1 degree. This correspondence table is determined by eliciting expert knowledge. In addition to a time factor *T,* it should be noted that only variations greater than or equal to zero are retained from the pooled node accumulating all the probable variations, since the final variation *BT* can only assume positive values.

Finally, a modified severity score *N'_{Si}* is determined by applying the table of conditional probabilities of the variations *BT* to the initial severity score *N_{Si}.* The modified degree of severity can be restricted by a maximum severity score that cannot be exceeded (for example, in this case 5, that is, the maximum of the scoring scale). Thus, for an initial severity score of 1, the probability of a variation of 5 will not result in a severity score of 6, but will be added to the probability of reaching the maximum severity score, i.e., 5.

A probability distribution is thus acquired for the various degrees of the scoring scale for the considered sign of ageing.

A modified image *Pm* representing said sign of ageing with a modified degree of severity can be generated for the most probable modified degree of severity, in this case, for a degree of severity of 3, for example. As mentioned above, it is also possible to generate a modified image for an average degree of severity, notably acquired by weighting with the probability distribution acquired for each variation of the initial degree.

The initial severity score of the sign of ageing and the impact and pooled data for the nodes can depend on the skin type of the user. To this end, the method can comprise an additional step of receiving this typology information in order to select the suitable assessment and simulation models.

A similar approach can be provided for the age of the user, with the difference being that only the statistical model and the predictive evolution data can be a function of an age range of the user; the assessment of the initial severity score preferably is not based on a model that is a function of the age of the user. However, the actual age of the user can be used to improve, enhance or confirm the scoring model that is used.

Thus, a dedicated evolution model can be provided for several age ranges, notably a model for the 30 - 40 age range, a model for the 40 - 50 age range, etc. It will then be advantageous to implement "age-continuous" models via an interpolation function allowing any age value to be accepted, for example, between 18 and 65 years of age, without needing to resort to age range models.

More specifically, an interpolation function is provided for weighting the acquired result with one or more models. For example, for a user aged 25, it will be possible to use a model designed for an 18 - 35 age range and to weight the prediction by the model designed for their target age, namely 40 years (25 years + 15 years), i.e., a model for the 35 - 50 age range, for example.

Indeed, the rate of ageing is slower between the ages of 18 and 35 than between the ages of 35 and 50. Using only the 18-35 model could result in the ageing being underestimated, while using only the 35-50 model could risk overestimating the ageing.

One solution is to apply both models to the user and to take a weighted combination of their results. The weights can be determined by virtue of an ageing function.

## Claims

1. Computer-implemented method for modelling and assessing the evolution of the signs of ageing (S1, S2, S3, S4) in a user, comprising the following steps of:
- receiving data corresponding to a set of pixels of an image (Pi) of a body area of a user generally showing at least one visible sign of ageing;
- assigning a severity score (Nsᵢ) to the visible sign of ageing of said body area;
- receiving data corresponding to personalised values adapted to the user for all or some of the ageing factors, *fᵢ*;
- generating a modified severity score (N'sᵢ) as a function of a value of at least one ageing factor (*fᵢ*, T) by adding an evolution variation (ΔNsi) to the initial severity score (Nsi), the method being **characterised in that** the evolution variation is determined from the values of the ageing factors, *fᵢ*, by applying at least one probabilistic model (M) to the values of the ageing factors, *fᵢ*, the probabilistic model (M) being a causal Bayesian network having the following features :
- for each input variable corresponding to an ageing factor *fᵢ*, the model presents a causal link to a node defining an impact *Iᵢ* representing a variation in the degree of severity for the considered factor;
- The impact *Iᵢ* assumes the form of a table of conditional probabilities associating each modality of the factor *fᵢ* with a variation in the degree of evolution ;
- The impact nodes *Iᵢ* are causally linked to pooled downstream nodes *Aij* aimed at grouping together the probabilities and variations by degree of severity of several factors until a total final variation *ΔN_{Si}* can be determined;
- each pooled node *Aij* is annotated with a table of conditional probabilities associating the variations in the degree of evolution that can result from the association of these factors.

2. Method according to Claim 1, **characterised in that** it comprises an additional step of generating a modified image (Pm) of the body area of the user with a visible sign of ageing corresponding to the modified severity score.

3. Method according to any one of Claims 1 to 2, **characterised in that** at least one ageing factor (T) is independent of the user.

4. System configured to implement a method according to any one of Claims 1 to 3, **characterised in that** it comprises at least one first data input configured to receive data corresponding to a set of pixels of a digital image (Pi), a second data input configured to receive at least one value of an ageing factor (fᵢ), and at least one processor configured for:
- assigning, on the basis of said received pixels, a severity score (Nsᵢ) of at least one visible sign of ageing (S1, S2, S3, S4);
- modifying said severity score as a function of the received value of the ageing factor;
- returning said modified severity score (N'si).

5. System according to Claim 4, **characterised in that** it comprises a digital photograph acquisition device communicating with the first data input.

6. System according to any one of Claims 4 or 5, **characterised in that** it comprises an image generator (GAN) configured to generate, from the received image data (Pi), a modified image (Pm) showing the modified severity score (N'sᵢ).

## Patentansprüche

1. Computerimplementiertes Verfahren zur Modellierung und Beurteilung der Entwicklung von Anzeichen der Alterung (S1, S2, S3, S4) bei einem Benutzer, umfassend die folgenden Schritte:
- Empfangen von Daten, die einem Satz von Pixeln eines Bildes (Pi) eines Körperbereichs eines Benutzers entsprechen, der im Allgemeinen mindestens ein sichtbares Anzeichen der Alterung zeigt;
- Zuordnen einer Schweregradbewertung (N_{Si}) zum sichtbaren Anzeichen der Alterung des Körperbereichs;
- Empfangen von Daten, die personalisierten Werten entsprechen, die für alle oder einige der Alterungsfaktoren *fᵢ* an den Benutzer angepasst sind;
- Erzeugen einer modifizierten Schweregradbewertung (N'_{Si}) in Abhängigkeit von einem Wert mindestens eines Alterungsfaktors (*fᵢ*, T) durch Addieren einer Entwicklungsvariation (ΔNsi) zu der anfänglichen Schweregradbewertung (Nsi), wobei das Verfahren **dadurch gekennzeichnet ist, dass** die Entwicklungsvariation aus den Werten der Alterungsfaktoren, *fᵢ*, bestimmt wird, indem mindestens ein probabilistisches Modell (M) auf die Werte der Alterungsfaktoren, *fᵢ*, angewandt wird, wobei es sich bei dem probabilistischen Modell (M) um ein kausales Bayessches Netz mit folgenden Merkmalen handelt:
- für jede Eingangsvariable, die einem Alterungsfaktor *fᵢ* entspricht, präsentiert das Modell eine kausale Verbindung zu einem Knoten, der eine Auswirkung *Iᵢ* definiert, die eine Variation des Ausmaßes des Schweregrads für den betrachteten Faktor repräsentiert;
- die Auswirkung *Iᵢ* nimmt die Form einer Tabelle von bedingten Wahrscheinlichkeiten an, die jede Modalität des Faktors *fᵢ* mit einer Variation des Entwicklungsgrads assoziieren;
- die Auswirkungsknoten *Iᵢ* werden kausal mit gepoolten nachgelagerten Knoten *Aij* verknüpft, um die Wahrscheinlichkeiten und Variationen nach dem Ausmaß des Schweregrads mehrerer Faktoren miteinander zu gruppieren, bis eine endgültige Gesamtvariation *ΔN_{Si}* bestimmt werden kann;
- jeder gepoolte Knoten *Aij* wird mit einer Tabelle von bedingten Wahrscheinlichkeiten annotiert, die die Variationen des Entwicklungsgrads assoziieren, die aus der Assoziation dieser Faktoren resultieren können.

2. Verfahren nach Anspruch 1, **dadurch gekennzeichnet, dass** es einen zusätzlichen Schritt des Erzeugens eines modifizierten Bildes (Pm) des Körperbereichs des Benutzers mit einem sichtbaren Anzeichen der Alterung entsprechend der modifizierten Schweregradbewertung umfasst.

3. Verfahren nach einem der Ansprüche 1 bis 2, **dadurch gekennzeichnet, dass** mindestens ein Alterungsfaktor (T) vom Benutzer unabhängig ist.

4. System, das dazu ausgelegt ist, ein Verfahren nach einem der Ansprüche 1 bis 3 zu implementieren, **dadurch gekennzeichnet, dass** es mindestens einen ersten Dateneingang, der dazu ausgelegt ist, Daten zu empfangen, die einem Satz von Pixeln eines digitalen Bildes entsprechen (Pi), einen zweiten Dateneingang, der dazu ausgelegt ist, mindestens einen Wert eines Alterungsfaktors (fᵢ) zu empfangen, und mindestens einen Prozessor umfasst, der ausgelegt ist zum:
- Zuweisen, auf Basis der empfangenen Pixel, einer Schweregradbewertung (N_{Si}) von mindestens einem sichtbaren Anzeichen der Alterung (S1, S2, S3, S4);
- Modifizieren der Schweregradbewertung als Funktion des empfangenen Werts des Alterungsfaktors;
- Ausgeben der modifizierten Schweregradbewertung (N'_{Si}).

5. System nach Anspruch 4, **dadurch gekennzeichnet, dass** es eine Digitalfotografie-Erfassungsvorrichtung umfasst, die mit dem ersten Dateneingang kommuniziert.

6. System nach einem der Ansprüche 4 oder 5, **dadurch gekennzeichnet, dass** es einen Bildgenerator (GAN) umfasst, der dazu ausgelegt ist, aus den empfangenen Bilddaten (Pi) ein modifiziertes Bild (Pm) zu erzeugen, das die modifizierte Schweregradbewertung (N'_{Si}) zeigt.

## Revendications

1. Procédé mis en œuvre par ordinateur de modélisation et d'évaluation de l'évolution des signes de vieillissement (S1, S2, S3, S4) chez un utilisateur, comprenant les étapes suivantes :
- la réception de données correspondant à un ensemble de pixels d'une image (Pi) d'une zone corporelle d'un utilisateur montrant en général au moins un signe visible de vieillissement ;
- l'attribution d'un score de sévérité (N_{Si}) au signe visible de vieillissement de ladite zone corporelle ;
- la réception de données correspondant à des valeurs personnalisées adaptées à l'utilisateur pour tout ou partie des facteurs de vieillissement, fᵢ ;
- la génération d'un score de sévérité modifié (N'_{Si}) en fonction d'une valeur d'au moins un facteur de vieillissement (fᵢ, T) en ajoutant une variation d'évolution (ΔNsi) au score de sévérité initial (NSi), le procédé étant **caractérisé en ce que** la variation d'évolution est déterminée à partir des valeurs des facteurs de vieillissement, fᵢ, en appliquant au moins un modèle probabiliste (M) aux valeurs des facteurs de vieillissement, fᵢ, le modèle probabiliste (M) étant un réseau bayésien causal présentant les caractéristiques suivantes :
- pour chaque variable d'entrée correspondant à un facteur de vieillissement fᵢ, le modèle présente un lien causal sur un nœud définissant un impact Iᵢ représentant une variation du degré de sévérité pour le facteur considéré ;
- l'impact Iᵢ se présente sous la forme d'une table de probabilités conditionnelles associant chaque modalité du facteur fᵢ à une variation du degré d'évolution ;
- les nœuds d'impact Iᵢ sont liés causalement à des nœuds aval regroupés Aij visant à regrouper les probabilités et variations par degré de sévérité de plusieurs facteurs jusqu'à ce qu'une variation finale totale ΔN_{Si} puisse être déterminée ;
- chaque nœud regroupé Aij est annoté avec une table de probabilités conditionnelles associant les variations du degré d'évolution pouvant résulter de l'association de ces facteurs.

2. Procédé selon la revendication 1, **caractérisé en ce qu'**il comprend une étape supplémentaire de génération d'une image modifiée (Pm) de la zone corporelle de l'utilisateur avec un signe visible de vieillissement correspondant au score de sévérité modifié.

3. Procédé selon l'une quelconque des revendications 1 et 2, **caractérisé en ce qu'**au moins un facteur de vieillissement (T) est indépendant de l'utilisateur.

4. Système configuré pour mettre en œuvre un procédé selon l'une quelconque des revendications 1 à 3, **caractérisé en ce qu'**il comprend au moins une première entrée de données configurée pour recevoir des données correspondant à un ensemble de pixels d'une image numérique (Pi), une deuxième entrée de données configurée pour recevoir au moins une valeur d'un facteur de vieillissement (fᵢ), et au moins un processeur configuré pour :
- l'attribution sur la base desdits pixels reçus d'un score de sévérité (N_{Si}) d'au moins un signe visible de vieillissement (S1, S2, S3, S4) ;
- la modification dudit score de sévérité en fonction de la valeur du facteur de vieillissement reçue ;
- la restitution dudit score de sévérité modifié (N'_{Si}).

5. Système selon la revendication 4, **caractérisé en ce qu'**il comprend un dispositif numérique d'acquisition de photographies communiquant avec la première entrée de données.

6. Système selon l'une quelconque des revendications 4 ou 5, **caractérisé en ce qu'**il comprend un générateur d'image (GAN) configuré pour générer, à partir des données d'image reçues (Pi), une image modifiée (Pm) représentant le score de sévérité modifié (N'_{Si}).
